# EUROPEAN PATENT APPLICATION

(11) **EP 1 637 188 A1**
(43) Date of publication of application: **22.03.2006**
(21) Application number: 04104019.7
(22) Date of filing: 20.08.2004
(51) Int. Cl.: A61Q 5/00, A61Q 5/12, A61Q 13/00, A61K 8/04, A61K 8/81, A61K 8/49, A61K 8/42, A61K 8/64, A61K 8/65

(54) **Improved liquid/sprayable compositions comprising fragranced aminoplast capsules**

(71) Applicant: FIRMENICH SA, 1211 Genève 8 (CH)
(72) Inventor: Holzner, Günter, 1211 Grand-Lancy (CH); Verhovnik, Glenn, 1224 Chene-Bougeries (CH)
(74) Representative: Salvaterra-Garcia, Maria de Lurdes

(57) **Abstract**

A product comprising a surfactant or a surface active ingredient on the one hand and a reactive perfumed capsule-containing base on the other hand, which further comprises a stabilising polymer system selected from AMPS polymers or copolymers and a combination of non-ionic with cationic polymers. This allows a prolonged storage of the perfuming capsules without alteration of the latter and without the need to shake the composition before use in order to obtain an homogeneous dispersion of capsules.

## Description

### Technical Field

The present invention relates to the perfume and consumer product industries. It concerns more particularly improved liquid and possibly sprayable compositions comprising aminoplast microcapsules containing fragrances, in particular fragranced melamine resin microcapsules, intended to be used in consumer products traditionally fragranced. The invention is characterised by the fact that the compositions comprise specific combinations of polymers capable of stabilising aqueous dispersions of aminoplast capsules when in the presence of potentially capsule-lysing surfactants. In low viscosity compositions, the specific combination of polymers is capable of keeping a homogeneous dispersion of the fragranced microcapsules thus avoiding the need to redisperse the microcapsules before use.

### Background Art

Melamine resins and urea resins constitute the most important representatives of the amino resins. They result from a kind of Mannich reaction between NH-containing compounds, nucleophilic molecules and carbonyl-containing compounds. NH-Components are mainly urea H₂N-CO-NH₂ or melamine (2,4,6-triamino-1,3,5-triazine). The carbonyl component is predominantly formaldehyde (rarely ketones or other aldehydes). Nucleophilic components may be H-acidic (halogen acids), OH compounds (alcohols, carboxylic acids), or NH compounds (urea, melamine, amines etc.) The resulting "aminoplastics" are colourless. Melamine resins are used for shatter-proof tableware. Moreover, the literature reports the use of amino resins for the encapsulation of active substances and mentions in particular the potential use of such encapsulation systems in perfumery and cosmetic applications. Therefore, amino resin based capsules, also commonly designated as aminoplast capsules, are the subject of a variety of literature reports and patent applications relating to the perfumery and cosmetic industries. In practice, these polymers are capable of forming a protecting shell around the active ingredient that one wishes to protect, thus providing an encapsulation system characterised by its water-insolubility. The active ingredient protected by the capsule may be released through mechanical rupture of the microcapsules, which become brittle when dry.

Now, while capsules based on the use of these polymers provide a good way of protecting volatile and labile ingredients such as perfuming compounds from degradation in certain media, for instance in an aqueous environment, they cannot be used in functional consumer products wherein the treating base of the product comprises a surfactant system, as they turn out to be unstable in such a medium. More particularly, it is known in the art that even if the shell of such microcapsules is not soluble in water, a cleaning solution such as a detergent solution, which contains surfactant compounds, washes out the fragrance contained in the capsules and leaves an empty shell after some weeks. Therefore, this type of chemically reactive aminoplast microcapsules cannot be used in consumer products containing a surfactant system.

Another type of microcapsules encounters the same problematic and is therefore submitted to the same limitation in its use. These are coacervate systems. Coacervation, also called aqueous phase separation, is a very well known technique for encapsulating hydrophobic liquids. A coacervation process allows to provide oil-containing microscopic or larger capsules, the encapsulating material being a gelled hydrophilic colloid that is impervious to the oil and deposited evenly and densely around the oil forming the nucleus. The encapsulating colloid material is a protein which may be complexed with another colloid having an opposite charge. Now, the products of simple or complex coacervation processes are unstable in a surfactant-containing environment, and this prevents their use in most consumer products of the detergent or cleaning type.

The process for the preparation of aminoplast microcapsules containing encapsulated fragrance is a well-known state of the art and is described in the patent literature, for example in US Patent 3,516,941, US Patent 4,976,961, DE Patent 198,33,347 (BASF), WO Patent 01/51197 (BASF) and US Patent 6,261,483 (BASF), GB Patent application 2073132 and WO 98/28396.

The use of such fragranced aminoplast capsules in liquid formulations for household and cosmetic applications is also well-known. For example, US Patent 5,188,754, assigned to Procter & Gamble, describes detergent compositions which contain perfumes in the form of friable microcapsules. US Patent 5,137,646, also to Procter & Gamble, describes the preparation and use of perfumed aminoplast particles which are stable in fluid compositions such as fabric softeners. However, this composition requires a two-step manufacturing process where the perfume is firstly solidified with a meltable polymer followed by grinding of the solidified perfume and coating with the aminoplast resin.

It is also described that cationic transfer agents drive the deposition of such aminopast capsules on fabric, skin and hair. This is of particular importance when such aminoplast capsules are used in liquid rinse-off formulations like laundry detergents, fabric conditioners, shampoos, rinse-off hair conditioners and body washes.

US Patent 4,234, 627, assigned to Procter & Gamble, discloses a liquid fragrance coated with an aminoplast shell further coated by a water insoluble meltable cationic coating in order to improve the deposition of capsules from fabric conditioners. In US 4,973,422 (P&G), from 1989, it was then further described that capsules with a cationic coating provide improved substantivity to the surface being treated, such as fabric treated with a fabric softener. The same idea was described in 1991 in US 5,185,155, assigned to Unilever, where the selection of cationic polymers was enlarged to water soluble polymers and the type of encapsulation was distinct from those in the state of the art at the time. Patent application US 20040071742, assigned to IFF, discloses a similar technology where the fragranced aminoplast capsules are coated with cationic starch or cationic guar. International patent application WO 03/002699, assigned to Colgate-Palmolive, describes fabric softening compositions where a cationic cross-linked polymer improves deposition of friable aminoplast microcapsules. The improved deposition of cationic microcapsules in rinse-off formulations is also generally disclosed in US Patent application 2003/0171246, assigned to BASF, and International patent application WO 01/62376, assigned to Henkel.

However, as previously mentioned, the major down-side of the use of aminoplast capsules in rinse-off applications is that the perfumed capsules are not stable in such liquid formulations. The perfume gets extracted out/off the capsules by surface active ingredients in the formulation. So far, none of the aminoplast capsules described heretofore remain stable for 2 months at 45°C in rinse-off formulations, i.e. under storage conditions that are encountered in many practical circumstances.

Some attempts have been made to increase the stability of such fragranced aminoplast capsules by modifying the capsules' membrane. For example, International patent application WO 02/074430, to Quest International, which outlines the above-mentioned stability problems of aminoplast capsules in aqueous surfactant-containing products, suggests a solution based on the use in the capsule shell of a second polymer comprising a polymer or copolymer of one or more anhydrides. It particularly describes improved stability in hair shampoo of aminoplast capsules prepared in the presence of ethylene(maleic anhydride) copolymer. While this solution improves the resistance of the capsules to degradation, it also prevents, as a consequence of the protection provided, the active ingredient, namely the perfume, from being easily released from the capsules. Therefore, these modified capsules require more energy than the original ones for the release of perfume, and this can constitute a drawback for their use in application. Moreover, the resulting stability after 1 month at 37°C is not sufficient for these applications.

On the other hand, WO 01/94001, assigned to Syngenta Ltd., mentions the possibility of having a solid permeable shell of a polymer resin having surface modifying compounds capable of reacting with isocyanate incorporated therein. While being based on the use of melamine resins, the latter shells have been modified so as to become permeable and, as a consequence, are susceptible of loosing the perfuming ingredients there-encapsulated through a diffusion process during the storage of the capsules.

Therefore, the solutions provided until now by the prior art are not satisfactory.

The Applicant has also previously addressed this issue in unpublished pending International patent application PCT/IB03/03601, filed August 13, 2003, wherein there is proposed a solution to the above-mentioned problem via the use of a special packaging system comprising two compartments, the fragranced aminoplast or coacervate capsules being lodged in a compartment which is separate from that which contains the surface-active ingredients commonly present in such rinse-off formulations. Although such a solution has proved to be a major improvement over previously described systems, because the fragranced aminoplast capsules have a lower density than other liquids which are generally used in liquid household and cosmetic formulations, the capsules may still tend to separate from the formulation. The same problem of separation applies when fragranced aminoplast capsules are added directly to liquid formulations where the density of the liquid is different from that of the encapsulated perfume. Such liquids include water, silicone oils, organic and mineral oils, alcohols, glycols and glycerine. It was observed that the lower the viscosity of the formulation is, the faster the capsules are likely to separate.

In order to obtain a homogeneous dispersion of the capsules in a liquid formulation, this formulation needs to be thickened until it becomes a gel or a cream. Such thickened formulations are less convenient to dose and can no longer be easily sprayed.

We have now discovered that by using specially selected, water soluble or dispersible polymers, dispersions of fragranced aminoplast or coacervate capsules can be stabilised in liquid formulations without reducing the flowability, spreading or sprayability of the dispersion.

We describe here particular selections of stabilising polymers that are advantageous to formulate sprayable dispersions of aminoplast and coacervate capsules.

### Disclosure of the Invention

A first object of the invention is a liquid and possibly sprayable aqueous dispersion of fragranced aminoplast or coacervate capsules which contains anionic polymers selected from the group consisting of the AMPS (Acrylamido methylpropane sulfonate) polymers or copolymers and combinations of non-ionic and cationic polymers. The present invention thus provides stabilised perfume compositions containing fragranced aminoplast microcapsules, namely melamine resin capsules, or fragranced coacervate capsules. The compositions of the invention make it possible to provide perfumed consumer products which are stable during storage even at high temperatures and which do not require separation of the aminoplast capsules from the surface-active components of such perfumed consumer liquid formulations.

By "aqueous" dispersion it is meant here a composition essentially water based, possibly containing up to 97 or 98% by weight of water, relative to the total weight of composition, and typically containing at least 50% by weight of water, but which may also contain other solvents compatible with the capsules such as for example ethanol.

Another object of the invention is a cosmetic or household liquid formulation comprising the above-mentioned dispersions of fragranced aminoplast or coacervate capsules.

A further object of the invention relates to a method for treating a surface such as a textile, or yet skin or hair, using a formulation as defined here-above, which method consists in the application of such liquid or sprayable formulations to the surface to be treated in the usual manner in which such formulations are generally applied.

Melamine resin and coacervate capsules both present many advantages when used as encapsulating systems for perfuming ingredients in application, i.e. in consumer products. First of all, when they break during use, for instance by friction when used in a shampoo or shower gel type application, a fresh fragrance burst is perceived. On the other hand, when especially treated, these capsules are susceptible of being efficiently laid down on a surface during their use in an application. Therefore, when a surface is treated with a product according to the invention, the perfumed capsules settle on said surface, typically a textile, the skin or yet hair, depending on the nature of the surfactant-containing product, and provide an efficient release, by simple mechanical action such as a friction on the surface, of the perfuming ingredients encapsulated therein. These effects were not optimally obtainable until now as these types of reactive capsules were altered by the surfactant-containing base during storage, before their possible use.

In a first embodiment of the invention, the fragranced capsules consist of melamine resin microcapsules. These microcapsules are conventionally prepared by a polycondensation process comprising emulsifying the ingredient to be encapsulated, namely a perfuming ingredient or composition, in an aqueous solution of a melamin/formaldehyde resin, and then hardening the thus formed microcapsules. Suitable capsules for the purpose of the invention are commercially available by manufacturers such as BASF (under the tradename of Micronal® ). Typically, these capsules will encapsulate from 20 to 85% by weight of perfume, relative to their total weight. Preferably, fragranced melamine resin capsules will be used in the form of a liquid aqueous suspension or dispersion. However, the capsules may also be used in the form of a dried powder, obtained after a drying treatment of a liquid suspension, e.g. via a spray-drying treatment carried out in a generally known manner.

In a second embodiment of the invention, the perfuming dispersion contains fragranced capsules prepared by a simple or complex coacervation process. Simple coacervation involves the use of a single protein to form a capsule wall as phase separation is taking place. The complex process designates the use of a second oppositely charged non-protein polymer to bring about phase separation. Both methods are widely practiced in commercial processes and have been well described in the literature, namely in textbook works such as Capsule Technology and Microencapsulation, Chapter 4, M. Gutcho, New Jersey, 1972. Typically, these capsules have a perfume load comprised between 20 and 85% by weight relative to their total weight.

In both cases the concentration of capsules in the aqueous suspension or dispersion of the invention will be typically comprised between 0.5 and 10% by weight, more preferably from 0.5 to 5% by weight, relative to the total weight of aqueous suspension.

The nature of the fragrance contained in the capsules is immaterial in the context of the invention, provided that it is compatible with the materials forming the capsules. It will be typically chosen as a function of the perfuming effect that is desired to achieve with the dispersion or consumer product of the invention, and it will be formulated according to current practices in the art of perfumery. It may consist of a perfume ingredient or composition. These terms can define a variety of odorant materials of both natural and synthetic origin, currently used for the preparation of perfumed consumer products. They include single compounds or mixtures. Specific examples of such components may be found in the current literature, e.g. Perfume and Flavor Chemicals by S. Arctander 1969, Montclair, N.J. (USA). These substances are well known to the person skilled in the art of perfumeing consumer products, i.e. of imparting an odor to a consumer product traditionally fragranced, or of modifying the odor of said consumer product.

Natural extracts can also be encapsulated into the system of the invention ; these include e.g. citrus extracts such as lemon, orange, lime, grapefruit or mandarin oils, or essentials oils of plants, herbs and fruits, amongst other.

In addition to the fragranced capsules, the perfuming dispersion of the invention may comprise optional ingredients such as antibacterial agents, cosmetic emollients, vitamins, cooling agents, softeners, lubricants, gloss enhancing agents or any other current active ingredient used in cosmetic or household applications, as long as the latter do not alter the capsules.

According to the invention, the aqueous dispersion of the capsules further comprises a polymeric thickening system. Such thickening polymers may be selected from the anionic polymers and copolymers which are known under the designation of AMPS polymers or from combinations of non-ionic polymers with cationic polymers.

Such systems of water soluble or water dispersible polymers stabilise the fragranced aminoplast capsules in liquid household and cosmetic formulations by preventing separation of the capsules, particularly in low viscosity formulations, without affecting the flowability, spreading capability or sprayability of the liquid formulation.

Anionic polymers appear to be the best stabilizers for liquid dispersions of anionic aminoplast capsules. The dispersions are stable even at low viscosity because they are stabilised by electrostatic charge. Repulsive action between equal charges supports the stability of the dispersion. However, many anionic polymers have such a high anionic charge that they are liable to extract perfume from the aminoplast capsules, in much the same way as surfactants do. In addition, anionic polymers are generally used for their fixing properties and therefore they can also cause a sticky feel on fabric, skin and hair. Examples of such anionic polymers comprise gum arabic, carboxymethylcellulose, (for example, Blanose® from Aqualon), anionic polyacrylates and alkylated copolymers (for example Carbopol® from Noveon and Salcare® AST from Ciba, Luviflex® Soft from BASF).

However, we have now found that a particular family of anionic thickening polymers can stabilise aqueous dispersions of aminoplast capsules without reducing the sprayability and spreading of the formulation and without providing a sticky feel on fabric, skin and hair. Such polymers are described in US patents nos. 6,645,476 and 6,506,833 and are generally known as AMPS polymers. They contain acrylamidopropylmethylenesulfonic acid crosslinked and partially neutralized to 50% with aqueous ammonia, sold under the name Hostacerin® AMPS by the company Clariant and copolymers of this polymer, sold under the name Aristoflex® AMPS and Aristoflex® HMB by the company Clariant. As it will become apparent from the examples presented further on, the use of these particular anionic polymers provides improvement of the capsules' stability in the dispersion and in the presence of surfactants.

In another embodiment of the invention, the thickening polymer system is a combination of non-ionic and cationic polymers.

Non-ionic polymers are known to efficiently stabilise aqueous dispersions of aminoplast capsules at high viscosity. However, the resulting high viscosity formulations can be difficult to spread and not easily flowable and sprayable. In addition, even when the viscosity has reached the state of a gel or cream, many non-ionic polymeric thickeners can become inefficient at high temperature causing some separation of the capsules at 45°C or higher temperatures. This is due to the fact that such non-ionic thickeners have a reversed temperature dependence and can turn into low viscosity at evelated temperature. The use of non-ionic polymers on their own is therefore not an optimal solution to the capsules' stability problem.

Typical examples of such non-ionic polymers thus used include guar gums, hydroxyalkyl cellulose derivatives (for example, Tylose® from Shin Etsu and Clariant, Klucel® HF from Aqualon and Natrosol® from Hercules), carrageenan (namely from Kelco), cellulose, starch, maltodextrines, polymeric sugar derivatives, xanthan, PVP/VA copolymers (namely Luviskol® VA from BASF).

The complex formation between anionic aminoplast capsules and cationic polymers is highly desired to drive the deposition of the capsules from rinse-off formulations onto the surfaces on which the latter are applied. Therefore, such cationic polymers are also known to be added to liquid dispersions of fragranced aminoplast or coacervate capsules.

Commonly used such cationic polymers comprise gelatine, quaternized hydrolisates of proteins (for example Gluadin® WQT from Cognis); polyquaternium polymers as cited in the CTFA Cosmetic Ingredient Dictionary, like cationic cellulose derivatives (Merquats® 100 and Ucare@ JR 30 M from Amerchol), cationic guars (Cosmedia® Guar from Cognis), quaternized guars (Jaguar® C-162 from Rhodia) cationic polyacrylates (Salcare® SC 60 and Salcare® Super 7 from Ciba, or Eudragit® RL 30D from Röhm), cationic acrylamides (Rheovis® CDE from Ciba), polyquaternized polymers (like Luviquat® Care from BASF), polyethylene imine (Lupasol® P from BASF), quaternized polysiloxanes and emulsion polymerized aminosilicones (Abil® Quat 3270 from Degussa, Formasil® 410 from General Electric).

However, when such cationic polymers are added to aqueous dispersions of aminoplast capsules, it is not uncommon to observe some agglomeration and separation of the capsules.

We have now found that combinations of non-ionic polymers and cationic polymers provide the best stability of the dispersion of aminoplast or coacervation capsules in liquid rinse-off formulations. The advantage of this embodiment of the invention is that the polymer combination does not cause agglomeration and separation of the capsules and does not have a negative impact on the deposition effect of the cationic polymer on the anionic aminoplast capsule.

The non-ionic polymer protects the aminoplast capsule from agglomerating with the cationic polymer and the latter keeps the aminoplast capsules well dispersed even in low-viscosity formulations.

With this combination of non-ionic and cationic polymers, the total viscosity of the liquid formulation can be kept very low, such that the formulation is easy to spray and spreads well on surfaces such as skin, hair and fabric, or household surfaces. This embodiment of the invention thus also improves on the embodiments which resort to the use of only one type of thickening polymer as previously known and this is apparent from the examples presented further on.

Typical concentrations of thickeners in the above embodiments of the invention vary from 0.1 to 5%. When combinations of non-ionic and cationic polymers are used, the relative proportions of the latter are comprised in a range of 5:1 to 1:5. Preferably, comparable weight amounts of non-ionic and cationic polymer shall be used.

The compositions of the present invention comprising fragranced aminoplast capsules, namely melamine resin capsules, or coacervates provide a solution to the problem of instability of the latter in the presence of surfactants, without requiring physical separation between the capsules and the surfactant-containing treating base. They are adapted to be advantageously used in consumer products for cleaning or treating surfaces such as skin or hair, textile, tiles or other such household surfaces, glass windows, etc, and are particularly useful for application via sprayable type such products.

The invention thus also concerns a cosmetic or household liquid and possibly sprayable consumer product comprising a dispersion of fragranced aminoplastic or coacervate capsules containing AMPS polymers or copolymers, or a combination of non-ionic and cationic polymers.

Within the framework of the invention, such consumer products are mainly formed of surfactant-containing liquid bases. The latter have a treating or cleaning activity, and may thus also be referred to as "treating bases". More particularly these "treating bases" designate a composition the formulation of which is specifically designed to provide a specific functional activity related to the treatment of a surface. Such an activity may consist for instance of a cleaning, purifying, detergent, softening, lubricating, gloss enhancing, bleaching or other activity adapted to the treatment of a household surface or for the treatment of skin or hair. The formulation of such treating bases comprises ingredients of varied nature and functions, depending on the treatment one wants to achieve with the product in question. Nowadays, the range of functional products and formulations has become so extensive and subjected to such frequent changes that a description based on a product-by-product approach and on the definition, for each case, of the composition of the treating base is impractical and would not be exhaustive anyway. However, a person skilled in the art is capable of choosing, without undue effort, suitable ingredients for the preparation of such a treating base designed for a particular purpose.

Many examples of such "treating bases" can be found in art textbooks and other literature, including the patent literature, relating to liquid consumer products commonly perfumed and which may in particular resort to the use of perfume capsules of the melamin type. Such examples of typical literature are namely cited in the introduction above, but it goes without saying that the consumer products according to the invention can assume any form or formulation desired, provided that they contain a surfactant system which rendered prior known dispersions of fragranced aminoplast capsules, namely melamine resin or coacervate capsules, unstable as far as the release of the perfume contained therein, or their stability in the dispersion, was concerned.

Therefore, the consumer product of the invention is a ready-to-use perfumed consumer product with a treating or cleaning activity, typically a hair leave-on or rinse-off preparation, a shampoo, a shower gel a liquid detergent for laundry or household, a liquid textile softener or yet a deodorant for laundry.

Its treating base is characterised by the presence in its composition of a surfactant or surface active ingredient system comprising at least one surfactant or surface active ingredient, in a proportion varying between 0.1 and 90% by weight relative to the total weight of the base. The surfactant system used can be anionic, cationic, non ionic as well as amphoteric. However, there will be usually used a mixture of these types of surfactants. For example, for shampoo or shower gel applications, a combination of lauryl sulfate or laurylethersulfate with an amphoteric surfactant of the betaine type is common. For textile softeners, pure cationic surfactants such as distearyldimethyl-ammoniumchloride or di-(alkyl) hydroxyethyl methylammonium methosulfate or combinations of such cationic surfactants with non-ionic surfactants like ethoxylated fatty alcohols are typically used. On the other hand, household cleaners are often based on linear alkylsulfates (LAS), eventually in combination with non-ionic ethoxylated surfactants. These surfactant systems are given by way of example and shall not be taken to limit the invention, the person skilled in the art being quite capable of extending the principles of the instant invention to other surfactant systems of current use without undue effort. It goes without saying that the nature of the surfactant is quite immaterial for the practising of the invention, the objective of which is to improve upon any consumer product comprising conventional fragranced melamine resin or coacervate capsules and a surfactant in which such capsules are not normally stable, i.e. any surfactant which causes partial or total extraction of the fragrance contained in such capsules when put in contact therewith. Any consumer product containing at least one such surfactant in an amount susceptible of causing such lysing of the perfume in the capsules is therefore encompassed by the instant invention and hereby included.

The consumer products of the invention can be packaged in any form which is current for the packaging of known such consumables. They can in particular be packaged in bags made of a water-soluble materials, in a single compartment or in generally known multiple compartment packages, such as those described for example in International patent application WO 2004/018319, assigned to Henkel, or in prior literature patent literature there-cited.

Although the dispersions of the invention do not require this, it is also clear that the consumer products of the invention can also be packaged as generally described in unpublished International patent application PCT/IB03/03601, assigned to the Applicant and hereby included by reference. The latter essentially describes a multicompartment package for a consumer product of the above-mentioned type wherein the dispersion of aminoplast or coacervate capsules and the surfactant-containing treating base can be lodged in separate compartments of the package, so as to optimise the storage stability. Typically, the package comprises two compartments of more-or-less comparable volume. The compartment with the capsules will typically contain aqueous dispersions of from 0.5% to 10% weight of capsules, relative to the total weight of the aqueous dispersion according to the instant invention and as generally described above.

The container may be made of various materials. Typically, it is made of plastics such as PET, OPP, PE or polyamide and including mixtures, laminates or other combinations of these.

Different embodiments may be considered as regards the physical separation of the compartments of the container.

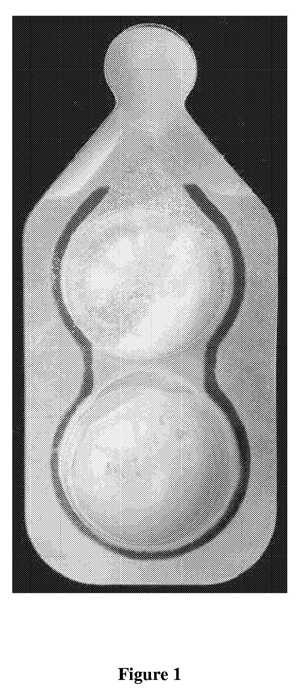

In a first embodiment, as represented in Figure 1, the separate compartments have a common joint formed by at least two sheets of polymeric material, welded or laminated with a breakable seal which ruptures internally along a longitudinal axis, without the external walls of the packing rupturing, when an external pressure is applied to one of the compartments. This embodiment enables to mix up the contents of the separated compartments just before use, after rupture of the internal portion of the joint separating said compartments. The sheet of polymeric material which ruptures internally along a longitudinal axis under the action of an external pressure is made of a polymeric material of the foam type and constitutes a breakable seal. Materials of this type include in particular polyethylene, polypropylene, polyamides, polyvinyl chloride and polyesters. These are preferably provided in the form of sheets having a thickness of between 0.005 and 0.2 mm. In this embodiment, the packaged product of the invention can be presented in the form of a mono-dose or single of 2 or more parts or blisters which are stored separately and mixed together prior to use, by applying a finger pressure on the outside of one blister to break the seal and mix the cleaning or treating base with the capsule dispersion of the invention.

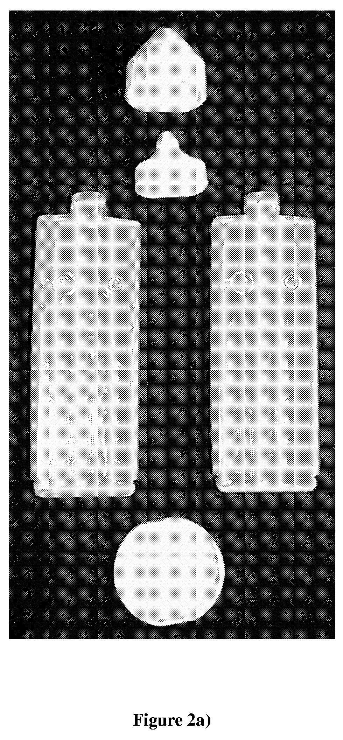

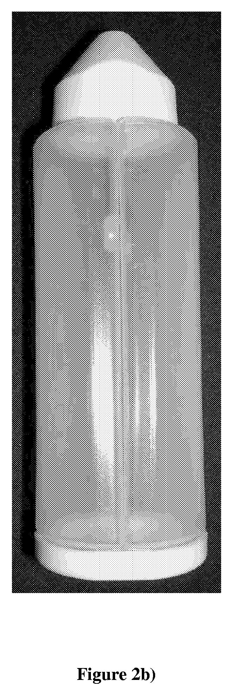

In a second embodiment of the package, illustrated in Figure 2a), the treating base on the one hand and the capsule dispersion on the other hand, are held in separate bodies, for instance having the shape of half cylinders, provided with a neck and an opening, and which can be arranged and fixed side by side as shown in Figure 2b). In this embodiment, there is no breakable seal between the two compartments, but the contents of the latter can simply be mixed before use, during outpouring, as the outlet piece unifies the two liquids coming from the separate compartments.

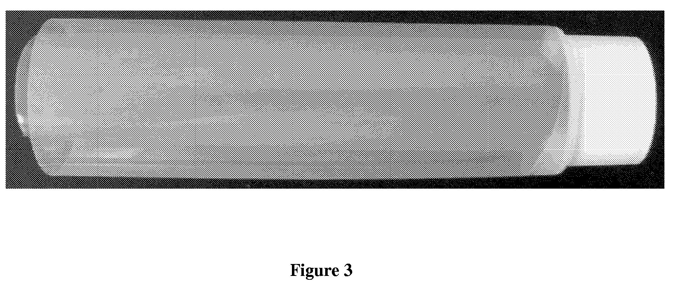

In another embodiment, as shown in Figure 3, the separate bodies are two cylinders forming a tube, the first one with a smaller diameter than that of the second one and located inside the second one, so that the outlets are formed by two concentric openings (of any shape) and allow the mixing of the contents of the compartments upon use of the product when the two liquids are squeezed out.

The packaged product can also be made of a water-soluble foil or film and have two compartments separated by a breakable seal, wherein the first component is a water-free liquid surfactant-containing base, and the second one is the invention's aqueous dispersion containing the reactive fragranced capsules. When used in an aqueous environment, the packaged product dissolves and the contents of the compartments are therefore combined upon use of the product.

The package is not limited to these embodiments, as many different ways of preparing two separated compartments assembled together in a way that allows their contents to be combined before use of the product may be considered and suit the packaging of the consumer product of the invention comprising a liquid and optionally sprayable aqueous dispersion of fragranced aminoplast or coacervate capsules and containing a stabilising polymer system selected from the group consisting of a) AMPS polymers or copolymers; and b) combinations of non-ionic and cationic polymers.

The invention will now be described in greater detail by way of the following examples wherein the temperatures are indicated in degrees Celsius and the abbreviations have the usual meaning in the art.

### Brief Description of the Drawings

Figure 1 illustrates a mono-dose or single dose packaged product according to the invention, wherein a first compartment (part 1) comprises fragranced capsules in the form of an aqueous suspension, while a second compartment (part 2) comprises a liquid detergent base. The upper and lower support of part 1 are made of a foam breakable seal while the lower support of part 2 is made of a solid un-breakable seal.
Figure 2a) illustrates a plastic bottle with separated compartments, wherein a first compartment (part 1) comprises fragranced capsules in the form of an aqueous suspension, while a second compartment (part 2) comprises a liquid detergent base.
Figure 2b) shows the closed bottle wherein the two compartments have been assembled together.
Figure 3 illustrates a plastic tube made of 2 cylindrical bodies, the first one (part 1) with a smaller diameter than the second one (part 2) and located inside the second one.

### Embodiments of the Invention

### Example 1

### Sprayable fabric freshener with AMPS polymers

The following compositions were prepared in a generally known manner with the following ingredients in the proportions indicated:

| Ingredients | I-A | I-B | I-C |
|---|---|---|---|
| | weight % | weight % | weight % |
| De-mineralised water | q.s. 100 | q.s. 100 | q.s. 100 |
| Hydroxypropyl beta-Cyclodextrine-(Kleptose® from Roquette) | 1.20 | 1.20 | 1.20 |
| Hydroxyethylcellulose-(Tylose® 60'000 P2 from Clariant) | 0.5 | | |
| Ammonium Acryloyldimethyltaurate / Beheneth-25 Methacrylate Copolymer (Aristoflex® HMB from Clariant) | | 0.5 | |
| Ammonium Polyacryloyldimethyltaurate (Hostacerin® AMPS from Clariant) | | | 0.5 |
| PEG-polysiloxane copolymer (Silwet® L-7600 from General Electric) | 0.1 | 0.1 | 0.1 |
| Diethylene Glycol | 0.1 | 0.1 | 0.1 |
| Propylene Glycol | 0.5 | 0.5 | 0.5 |
| Preservative (Kathon® CG from Rohm / Haas) | 0.1 | 0.1 | 0.1 |
| citric acid | till pH = 4 | till pH = 4 | till pH = 4 |
| 40% Aqueous dispersion of anionic aminoplast capsules containing 30% of encapsulated perfume (See for example WO 01/51197) | 1.0 | 1.0 | 1.0 |
| Viscosity by Brookfield | 162 mPa | 280 mPa | 204 mPa |
| | | | |
| Stability after 3 months at 45°C | separation | stable | stable |

The aminoplast capsules remained well dispersed in the liquid fabric freshener formulation and no perfume was extracted during ageing for 3 months at 45°C. When the formulation was sprayed onto fabric, the dry fabric released fragrance by rubbing on it. The rubbing action broke the friable aminoplast capsules provided a refreshing fragrance burst.

### Example 2

### Commercial sprayable fabric refreshener stabilised with AMPS polymers

0.5% By weight of Hostacerin® AMPS, relative to the total weight of product, were mixed into a commercial product typically used to spray dry fabrics for freshening of the latter against malodor or wrinkling, containing a non-ionic surface active ingredient system. After addition of Hostacerin® AMPS, the viscosity of the commercial fabric refresher increased from 5 mPa to 200 mPa and the composition remained well sprayable by a standard pump spray system. To the thus stabilised formulation, there were added 1% by weight, relative to the total product weight, of anionic aminoplast capsules (40% dispersion as described in WO 01/51197) and mixed thereto. The resulting dispersion remained homogeneous and stable upon storage for 3 months at 45°C. When the formulation was sprayed onto fabric, the dry fabric released fragrance by rubbing on it. The rubbing action broke the friable aminoplast capsules provided a refreshing fragrance burst.

### Example 3

### Sprayable leave-on hair refresher

The following compositions were prepared in a generally known manner with the following ingredients in the proportions indicated:

| Ingredients | III-A | III-B |
|---|---|---|
| | weight % | weight % |
| De-mineralised water | q.s. 100 | q.s. 100 |
| Ethanol | 10.0 | 10.0 |
| Ammonium Polyacryloyldimethyltaurate - 2% aqueous dilution (Hostacerin® AMPS from Clariant) | | 25.0 |
| Preservative (Liquapar® Optima from ISP) | 0.1 | 0.1 |
| 40% Dispersion of anionic aminoplast capsules containing 30% of encapsulated perfume (as described in WO 01/51197) | 1.0 | 1.0 |
| Liquid perfume | 0.15 | 0.15 |
| Viscosity by Brookfield | 6 mPa | 16 mPa |
| | | |
| Stability after 3 month at 45°C | separation | stable |

When the formulation III-B was spray-applied on dry hair, a strong fragrance burst was noticed on the dried hair after rubbing which caused fragrance release from the friable aminoplast capsules.

### Example 4

### Cationic concentrate of fragranced aminoplast capsules

A) Anionic melamine / formaldehyde capsules containing encapsulated liquid fragrance were prepared following the procedure described in Example 2 of International patent application WO 01/51197, the contents of which are hereby included by reference. The resulting dispersion of 40% of cationic aminoplast capsules had an average particle size of 3 - 10 microns. Upon ageing at ambient temperature for 1 month the particles agglomerated on the surface forming a hard layer due to absence of a non-ionic thickening agent.
B) Anionic melamine / formaldehyde capsules containing encapsulated liquid fragrance were prepared following the procedure described in Example 2 of International patent application WO 01/51197. After addition of melamin during hardening of the capsules, the capsules were coated with a cationic polymer by adding 1.25% of Ucare® JR 30 M (Polyquaternium-10 from Amerchol) to the dispersion and the reaction was finished as described in the procedure reported in the above-mentioned document. The resulting dispersion of 40% of cationic aminoplast capsules had an average particle size of 100 microns and became a hard gel within several days at room temperature. This gel was no longer dispersible in water.
C) Example B was repeated, but after the addition of the cationic polymer Ucare® JR 30 M, 0.4% by weight of Tylose® 200'000YP2 (Hydroxyethylcellulose from Clariant) were added to the dispersion. The reaction was completed as described in the procedure. The resulting dispersion of 40% by weight of aminoplast capsules remained fluid upon storage of 2 months at 45°C and could be easily dispersed in water. Average particle size was measured to be 30 microns.

Composition of example A), in the absence of the cationic polymer, provided particles with an average size of 3-10 microns, which shows that the addition of the cationic polymer causes agglomeration of the melamine/formaldehyde capsules, which could however be controlled and contained by the addition of the non-ionic thickener Tylose®.
When 1% of the above cationic concentrate of aminoplast capsules described in C) were added to a commercial hair shampoo comprising as surfactants Sodium Laureth Sulfate, Cocamidopropyl Betaine and Cocamide DEA, the capsules remained well on hair that was washed with this shampoo. After rubbing on the dried hair, a strong fragrance burst was noticed.
After ageing the shampoo containing capsules of example C) for 3 months at 45°C, there was still a fragrance burst noticed on hair treated with this shampoo.
1% of anionic concentrate of aminoplast capsules of example A) were added to the same shampoo, and the shampoo was applied on hair without rinsing. After rubbing on the dried hair, a strong fragrance burst was noticed. When the hair was rinsed with water, no fragrance burst was noticed after rubbing on the dried hair.
After ageing for 3 months at 45°C, there was no fragrance burst noticed on the dry hair, treated with this shampoo without rinsing. The encapsulated fragrance was washed out completely from the capules by the surfactants of the shampoo.

### Example 5

### Liquid rinse-off laundry deodorant

The following compositions were prepared in a generally known manner with the following ingredients in the proportions indicated:

| Ingredients | V-A | V-B | V-C |
|---|---|---|---|
| | weight % | weight % | weight % |
| De-mineralised water | q.s. 100 | q.s. 100 | q.s. 100 |
| Acrylamidopropyltrimonium chloride / Acrylamide copolymer - 2% aqueous dilution (Salcare® SC60 from Ciba) | 40.0 | 40.0 | 40.0 |
| Hydroxyethylcellulose - 1% aqueous dilution (Tylose® 60'000 from Clariant) | | 10.0 | 10.0 |
| Preservative (Kathon® CG from Rohm / Haas) | 0.1 | 0.1 | 0.1 |
| 40% dispersion of anionic aminoplast capsules containing 30% of encapsulated perfume ( as described in WO 01/51197) | 4.0 | 4.0 | 4.0 |
| Formasil® 410 (35% emulsion polymerised aminosilicone from General Electric Specialty Materials) | | | 1.0 |
| liquid perfume | 0.7 | 0.7 | 0.7 |
| viscosity by Brookfield | 1200 mPa | 1404 mPa | 576 mPa |
| stability after 3 months at 45°C | separation | stable | stable |

When 20 ml of composition V-B or V-C were added to the dispenser box of a washing machine loaded with 3.5 kg of laundry, the aminoplast capsules deposited well on the fabric during the rinse cycle. On the dry fabric a strong fragrance burst was noticed after rubbing the textile.
Similar deposition of aminoplast capsules could be obtained when the formulation of Example V-B was mixed 1:1 with a commercial fabric softener, comprising a surfactant of the cationic esterquat type, and was then dosed by the dispenser of the washing machine.
The stability of this product was also tested in a two compartment package as described before, wherein the capsule dispersion here-described was stored in on compartment and the commercial fabric softener base was lodged in the other compartment. The product remained stable for many months on the shelves.

### Example 6

### Liquid rinse-off hair conditioner

The following compositions were prepared in a generally known manner with the following ingredients in the proportions indicated:

| Ingredients | VI-A | VI-B |
|---|---|---|
| | weight % | weight % |
| De-mineralised water | q.s. 100 | q.s. 100 |
| Acrylamidopropyltrimonium chloride / Acrylamide copolymer - 1% aqueous dilution (Salcare® SC60 from Ciba) | 40.0 | 40.0 |
| Hydroxyethylcellulose - 2% aqueous dilution (Klucel® HF from Aqualon) | | 20.0 |
| Preservative (Liquapar® Optima from ISP) | 0.1 | 0.1 |
| 40% Dispersion of anionic aminoplast capsules containing 30% of encapsulated perfume (WO 01/51197) | 1.0 | 1.0 |
| Liquid perfume | 0.15 | 0.15 |
| Viscosity by Brookfield | 422 mPa | 707 mPa |
| | | |
| Stability after 3 months at 45°C | separation | stable |

When the formulation VI-B was applied on wet hair and rinsed-off, good deposition of the fragranced aminoplast capsules could be obtained. After rubbing on the dried hair, a strong fragrance burst was noticed.
Similar deposition of aminoplast capsules could be obtained when this formulation VI-B was mixed 1:1 with a commercial hair shampoo comprising as surfactants Sodium Laureth Sulfate, Cocamidopropyl Betaine and Cocamide DEA and was then applied on hair.
The stability of this product was also tested in a two compartment package as described before, wherein the capsule dispersion here-described was stored in on compartment and the commercial fabric softener base was lodged in the other compartment. The product remained stable for many months on the shelves.

## Claims

1. A composition in the form of a liquid and optionally sprayable aqueous dispersion of fragranced aminoplast or coacervate capsules, which contains a stabilising polymer system selected from the group consisting of
a) AMPS polymers or copolymers;
and
b) combinations of non-ionic and cationic polymers.

2. A composition according to claim 1, having a viscosity of between 1 and 10.000 mPa, preferably between 10 and 2.000 mPa, and more preferably between 10 and 200 mPa.

3. A composition according to claim 1, comprising anionic aminoplast capsules.

4. A composition according to claim 1 wherein the stabilising polymers are hydrophylic and water soluble or water dispersable.

5. A composition according to claim 1, further comprising a water dispersible or water soluble silicone compound, in an amount sufficient to significantly reduce the viscosity of said composition.

6. A composition according to claim 5, wherein said silicone compound is present in an amount of 0.1 to 10% by weight, relative to the weight of the composition.

7. A composition according to claim 1, which contains from 0.5 to 10% by weight of fragranced capsules, relative to the weight of the composition.

8. A composition according to claim 1, wherein the polymer system is present in a weight range of from 0.1 to 5% by weight, relative to the weight of the dispersion.

9. A composition according to claim 1, in the form of a cosmetic or household consumer product.

10. A consumer product according to claim 9, selected from the group consisting of a liquid detergent, a fabric softener, a shampoo, a liquid soap, a shower gel, a liquid all-purpose cleaner, a sprayable fabric freshener or a hair spray freshener.

11. A consumer product according to claim 10, containing from 0.5 to 90% weight, relative to the weight of the product, of a surfactant system or surface active ingredient system.

12. A consumer product according to claim 11, conditioned in package comprising physically separated compartments, wherein the surfactant system and the dispersion of fragranced aminoplast or coacervate capsules are lodged in distinct compartments.

13. A consumer product according to claim 12, comprising two compartments having a common joint formed by at least two sheets of polymeric material, welded or laminated with a breakable seal which ruptures internally along a longitudinal axis without the external walls of the package rupturing, when an external pressure is applied to one of the compartments.

14. A consumer product according to claim 12, having two compartments, each comprising an opening located close to one another in a manner such as to allow unification of their contents upon use.

15. A consumer product according to claim 12, having two physically separated compartments made of a water-soluble foil or film and which are physically separated by a breakable seal.

16. A method for treating a surface using a product according to any one of claims 9 to 15, which consists in applying said product onto the surface to be treated, preferably via spraying.

17. A method for treating a surface using a product according to any one of claims 12 to 15, comprising the steps of mixing the contents of the physically separated compartments of the package and applying the combined contents of the components onto the surface to be treated.

18. A method according to claim 17, using a packaged product according to claim 10, wherein the contents of the separate compartments are combined by applying pressure on the outside of one compartment to induce the breaking of the seal separating the compartments.
